# EUROPÄISCHE PATENTSCHRIFT

(11) **EP 2 425 244 B1**
(45) Veröffentlichungstag und Bekanntmachung des Hinweises auf die Patenterteilung: **01.05.2013**
(21) Anmeldenummer: 10712027.1
(22) Anmeldetag: 09.03.2010
(51) Int. Cl.: G01N 33/50, B01J 4/02, B01J 19/00, B01L 3/00, B41J 2/175, C12M 1/18

(54) **KARTUSCHE UND BETRIEBSVERFAHREN FÜR REAGENZIEN EINES BIOSENSORSYSTEMS**
CARTRIDGE AND OPERATING METHOD FOR REAGENTS OF A BIOSENSOR SYSTEM
CARTOUCHE ET PROCÉDÉ DE FONCTIONNEMENT POUR DES RÉACTIFS D'UN SYSTÈME BIOCAPTEUR

(30) Priorität: 30.04.2009 DE 102009019650
(43) Veröffentlichungstag der Anmeldung: 07.03.2012
(73) Patentinhaber: Siemens Aktiengesellschaft, 80333 München (DE)
(72) Erfinder: BARLAG, Heike, 90408 Nürnberg (DE); OSTERMAIER, Jochen, 91056 Erlangen (DE)
(86) Internationale Anmeldenummer: PCT/EP2010/052969
(87) Internationale Veröffentlichungsnummer: WO 2010/124895

(56) Entgegenhaltungen:
- WO-A2-00/56455
- WO-A2-02/090995
- US-A- 5 075 082

## Beschreibung

Die Erfindung betrifft eine Kartusche, welche mit Fluiden, insbesondere Reagenzien, für den Betrieb von Biosensorsystemen zur Messung von insbesondere Enzym-Inhibitoren zur Kontrolle der Wasserqualität eingesetzt wird. Weiterhin wird ein Verfahren zum Betrieb der Kartusche am Biosensors beschrieben.

Allgemein ist der Einsatz von Biosensoren bekannt, die in der Regel organische Stoffe erfassen können. Der zentrale Biosensor trägt in der Regel ein bestimmtes Enzym. Gemessen werden allgemein katalytische Aktivitäten, wobei die Messungen der katalytischen Aktivitäten zwischen einer Messung mit einer Probe und einer Messung ohne Probe ausgewertet wird. Da bei einem Biosensor Enzyme, also Proteine, verwendet werden, ist der Einsatz dieser Biosensorsysteme damit verbunden, dass bestimmte Betriebsmittel eine endliche Haltbarkeit aufweisen. Die Versorgung eines Biosensorsystems erfordert somit eine Reihe von Reagenzien. Diese Reagenzien werden beim Betrieb des Biosensorsystems verbraucht und müssen von Zeit zu Zeit aufgefüllt werden.

Wird ein sogenanntes "wartungsarmes" Gerät gewünscht, so soll eine derartige Wiederauffüllung, beispielsweise nicht häufiger als einmal pro Woche, notwendig sein. Dies bedeutet, dass in der Kartusche enthaltene Reagenzien derart behandelt werden müssen, dass sie mindestens eine Woche stabil sind.

Die für einen Biosensor in einem Biosensorsystem notwendigen Reagenzien liegen meist in flüssiger Form vor, so dass eine Ankopplung an ein Biosensorsystem in der Regel über die flüssige Phase geschieht. Reagenzien werden bisher einzeln angemischt und in entsprechende Behälter abgefüllt.

Der Erfindung liegt die Aufgabe zugrunde, eine Kartusche bereitzustellen, mittels der sämtliche Flüssigkeiten zum Betrieb eines Biosensorsystems verschlossen bevorratet werden können und das Volumen der jeweiligen in einem Behälter der Kartusche enthaltenen Reagenzien durch Anpassung der Behältergröße optimiert ist, so dass die Reagenzien restlos verbraucht werden. Weiterhin ist ein Betriebsverfahren zum Einsatz einer derartigen Kartusche anzugeben, wobei Wasser freie Reagenzien zu schützen sind.

Die WO 00/56455 offenbart eine Kassette, enthaltend mehrere miteinander verbundene Reagenzienbehälter, deren offene Oberseite mit einer durchstoßbaren Metallfolie verschlossen ist, und deren Boden zentralsymmetrisch schräg abfällt. Die beschriebene Kassette wird zur Reagenzienbereitstellung bei z.B. Immunoassays verwendet.

Die WO 02/090995 offenbart eine Kartusche, enthaltend mehrere miteinander verbundene Reagenzienbehälter, deren offene Oberseite mit einer durchstoßbaren Folie verschlossen ist, wobei der Boden mindestens einiger Behälter schräg verläuft. Die Kartusche ist an eine Reagenzienentnahmevorrichtung ankoppelbar.

Die Lösung dieser Aufgabe geschieht durch die Merkmalskombination der jeweiligen Hauptansprüche. Vorteilhafte Ausgestaltungen sind den Unteransprüchen zu entnehmen.

Die Erfindung basiert darauf, dass der die Volumina der Reagenzien und der entsprechenden Behälter je nach spezifischem Verbrauch optimiert sind. Gleichzeitig hat eine Kartusche mit mehreren Behältern bereits sämtliche Flüssigkeiten bzw. Reagenzien zum Betrieb eines Biosensorsystems verschlossen bevorratet, mit dem Vorteil, dass diese bei der Lagerung der Kartusche in wässrigem oder Wasser freiem Zustand vorliegen können. Durch die unterschiedlichen Volumen der Behälter in einer Kartusche können die beim Betrieb des Biosensors mit unterschiedlichem spezifischem Verbrauch behafteten Reagenzien derart bereitgestellt werden, dass eine vollständige Entleerung der einzelnen Behälter für eine vorgegebene Zeit, beispielsweise für eine Woche, möglich ist. Entsprechend werden die einzelnen Behälter der Kartusche in ihrem Volumen nach dem spezifischen Verbrauch der Reagenzien angepasst.

Die Behälter weisen einen zur Waagerechten schrägen Boden auf, wobei die Ankopplung an das Biosensorsystem über Hohlnadeln geschieht, die im angekoppelten Zustand durch die Folie durchgestochen sind und deren Ende im tiefsten Bereich des schrägen Bodens positioniert ist.

Durch die Einbringung einer zusätzlichen Vertiefung im tiefsten Bereich des schräg abfallenden Bodens eines jeden Behälters, beispielsweise in Form einer halbkugelförmigen Vertiefung lässt sich in vorteilhafter Weise die Ausnutzung der einzelnen Reagenzien beim Betrieb des Biosensors bis zu einer geringen Restmenge betreiben.

Vorteilhaft ist eine Belüftung der einzelnen Behälter zum Druckausgleich, indem neben einer Öffnung für Hohlnadeln zur Entnahme von Reagenzien, eine Öffnung in die Folie, am gleichen Behälter angebracht wird, die zur Belüftung des Behälters dient.

Die Belüftung kann insbesondere mit einem Wasser freien Gas betrieben werden, so dass Reagenzien, die Hydrolyse empfindlich sind, beispielsweise mit trockener Luft versorgt werden. Es kann jedes Gas, welches kein Wasser enthält, verwendet werden.

Der Aufbau von abgeschlossenen Volumen, der durch Behälter einer Kartusche realisiert ist, sieht die oberseitige Begrenzung und Abdichtung der Behälter durch eine Folie vor. Damit sind biologische Reagenzien und allgemein Hydrolyse empfindliche Substanzen in den Behältern Wasser frei gelagert werden.

Es ist sehr vorteilhaft, das Trocknungsmittel direkt auf der Kartusche, zur Darstellung von Wasser freiem Gas.

Im Folgenden werden anhand von schematischen die Erfindung nicht einschränkenden Figuren Ausführungsbeispiele beschrieben.
- Figuren 1, 1A, 1B: zeigen die Aufsicht und die Seitenansicht einer Kartusche 10 mit den Umrissen der einzelnen Behälter 11-17,
- Figuren 2, 2A, 3,3A, 4,4A: zeigen jeweils die Aufsicht der Kartusche und eine Schnittdarstellung entsprechend Schnitt A-A, bei einer Belüftung mit trockenem Gas,
- Figuren 5, 5A, 6, 6A,: zeigen jeweils die Aufsicht der Kartusche und eine Schnittdarstellung entsprechend Schnitt A-A, bei einer langfristigen Lagerung von Reagenzien.

Eine Kartusche 10 mit mehreren Behältern 11-17, enthält sämtliche Flüssigkeiten, insbesondere Reagenzien, zum Betrieb eines Biosensors in einem Biosensorsystem. Der Vorteil einer Lösung entsprechend der Erfindung besteht darin, dass die Volumenverhältnisse der einzelnen Behälter dem Verbrauch beim Betrieb des Biosensors angepasst sind und so eine vollständige Entleerung zu einer vorgegebenen Zeit von sämtlichen der Behälter gleichzeitig möglich.

Die Hohlnadeln 5 sind starr mit dem Biosensorsystem verbunden. Damit wird vermieden, dass die Schläuche an der rückwärtigen Seite der Hohlnadeln 5 bewegt werden und damit Störungen, beispielsweise durch Knicken der Schläuche auftreten. Durch das Knicken der Schläuche ermüdet deren Material und deren Innenvolumen verändert sich, sodass nach dem Anschließen der Kartusche Luft in den Schläuchen auftauchen kann.

Eine wasserfreie Belüftung von einzelnen Behältern ermöglicht die Lagerung von Hydrolyse empfindlichen Reagenzien in wasserfreien organischen Lösungsmitteln.

Die Lagerung von einigen Reagenzien in der Kartusche entsprechend der Erfindung kann damit ohne Kühlung betrieben werden.

Zur Belüftung mit wasserfreier Luft kann beispielsweise ein Wasserabsorber vorgeschaltet sein, sodass Behälter mit Umgebungsluft gefüllt werden können, die in einer Vorbehandlung getrocknet ist.

Zur Erzielung der insgesamt möglichst restlosen Entleerung aller Behälter gleichzeitig sind die Enden der Hohlnadeln 5 auf der Seite eines Behälters jeweils in der Nähe einer Vertiefung 18 positioniert, die sich im Boden der Behälter befindet. Der Boden der einzelnen Behälter ist jeweils schräg angestellt.

Die Reagenzien, die zu bevorraten und dem Betrieb des Biosensors zur Verfügung zu stellen sind, werden in einer Kartusche eingefüllt. Die Anzahl der Behälter entspricht in der Regel der Anzahl der Reagenzien. Die Behältergröße ist derart gewählt, dass nach einer definierten Messzeit, wie beispielsweise eine Woche, möglichst keine Reste in der Kartusche in irgendeinem Behälter vorhanden sind. Die Volumenverhältnisse sind dabei schematisch durch die unterschiedlich großen Umrisse der einzelnen Behälter dargestellt. Berücksichtigt ist der spezifische Verbrauch der Reagenzien aus den einzelnen Behältern.

Dabei kann durch die Folie in die einzelnen Kammern gestochen werden, womit eine fluidische Ankopplung an das Biosensorsystem hergestellt wird.

Um durch den Betrieb des Biosensorsystems bzw. der Entnahme von Reagenzien aus den Behältern keinen Unterdruck innerhalb eines Behälters zu erzeugen, und damit ein Blockieren der Flüssigkeitsentnahme zu verursachen, werden zur Belüftung von Behältern beim Ankoppeln der Kartusche Öffnungen bzw. Löcher in die Folie gestanzt.

Speziell zur Belüftung von einzeln Behältern, in denen wasserfrei zu lagernde Reagenzien enthalten sind, wird eine Belüftung darstellt, in Kombination, beispielsweise mit einem Element zur Versorgung mit trockenem Gas, wie trockener Luft. Alternativ können auch wasserfreie Gase zur Spülung entsprechender Behälter eingesetzt werden.

Die Reagenzien liegen vorzugsweise als Fluide vor und können beispielsweise als chemische Puffer in drei verschiedenen Zusammensetzungen wie Salzsäure, Mischung aus verschiedenen Enzymen und zwei verschiedene wasserlösliche Lösungen von Enzymsubstraten bereitgestellt sein. Die wasserfreien Lösungen der Enzymsubstrate werden relativ zeitnah vor dem Gebrauch im Biosensorsystem mit Pufferlösungen gebrauchsfertig angemischt, da diese Hydrolyse empfindlich sind. Die wasserfreien Stammlösungen dürfen daher auch nicht mit feuchter Luft in Kontakt kommen. Die entsprechenden Behälter müssen zur Aufnahme der entsprechenden Reagenzien daher mit getrockneter Luft oder anderen trockenen Gasen belüftet werden.

Mit der Erfindung können chemische Puffer und die Enzyme trocken in der Kartusche bevorratet werden. Dadurch werden diese besonders haltbar, sodass die Kühlung bei der Lagerung entfallen kann. Nach dem Anschluss der Kartusche an ein Biosensorsystem werden die einzelnen Behälter mit den trocken gelagerten Reagenzien mit Reinstwasser befüllt. Dadurch werden diese Reagenzien gebrauchsfertig und die Kartusche ist mit allen Reagenzien einsetzbar.

Für den Einsatz des Biosensorsystems bzw. der Kartusche an besonders warmen Einsatzorten kann die Kartusche gekühlt werden. Hier ist der Einsatz einer Metallplatte unterhalb der Kartusche in Kombination mit einem Peltierelement möglich, welches diese Metallplatte kühlt. Die Metallplatte kann speziell geformt sein, sodass die thermisch empfindlichen Reagenzien besonders gut gekühlt werden.

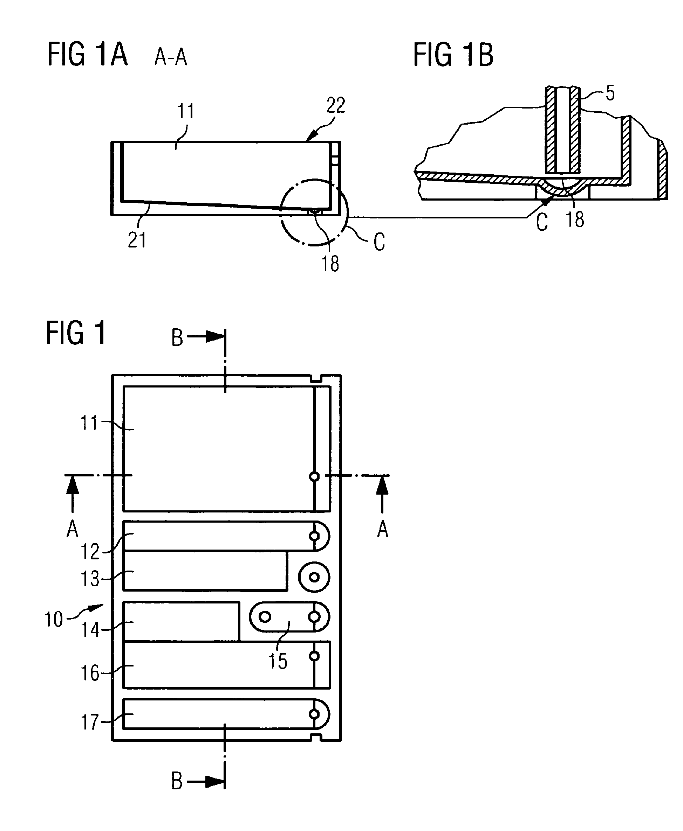

Zu den Figuren 1, 1A und 1B ist anzumerken, dass in verschiedenen, teilweise geschnittenen Ansichten, eine Kartusche von verschiedenen Betrachtungsrichtungen und in Einzelheiten dargestellt ist. Figur 1 zeigt eine Kartusche 10 in der Aufsicht, wobei unterschiedlich große Behälter 11-17 in ihren Umrissen erkennbar sind. Weiterhin sind durch Kreise die Punkte gekennzeichnet, an denen Hohlnadeln 5 durch eine die Behälter von oben abdeckende Folie durchgestochen werden. An diesen in Figur 1 sichtbaren Kreisen befinden sich die Vertiefungen 18 des Bodens der Kartusche, wobei die in den Behältern 11-17 von oben eingeführten Hohlnadeln 5 mit ihren Enden, im angekoppelten Zustand der Kartusche an ein Biosensorsystem in der Nähe der Vertiefung 18 positioniert sind.

Figur 1A zeigt den Schnitt A-A aus Figur 1, wobei der schräg verlaufende Boden des Behälters 11 die Vertiefung 18 aufweist. Diese zeigt eine etwa halbkugelförmige Ausbildung. Der gleiche Gegenstand ist in der Einzelheit A in Fig. 1B nochmals angeführt.

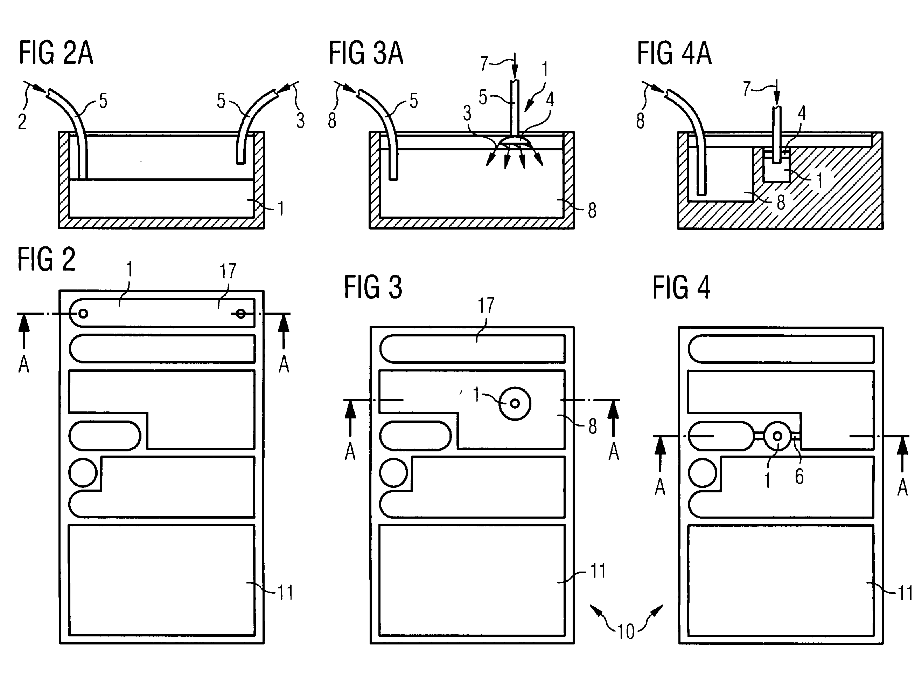

Fig. 2 und Fig. 2A zeigen eine Variante mit Trocknungsmittel 1 auf der Kartusche im Behälter 17. Es kann ein getrocknetes also Wasser freies Gas erzeugt werden und zur Belüftung von Behältern bei der Entnahme von Reagenzien aus diesen eingeleitet werden.

Die Figuren 3 und 4 mit den zugehörigen Figuren 3A und 4A zeigen in der Aufsicht und in der jeweiligen Schnittansicht ebenfalls Belüftungsmöglichkeiten, wobei getrocknete Luft 3 in mehreren Varianten erzeugt und den bestimmten Behältern zugeführt wird. In Fig. 3A wird die Belüftung 7 auf das Trocknungsmittel 1 geleitet und hindurchgeführt, so dass getrocknete Luft 3 entsteht.

In Fig. 4A wird eine Membran 4 eingesetzt, die ein Vermischung von Reagenzien und Trocknungsmittel verhindert, jedoch eine Trocknung von Gas erlaubt.

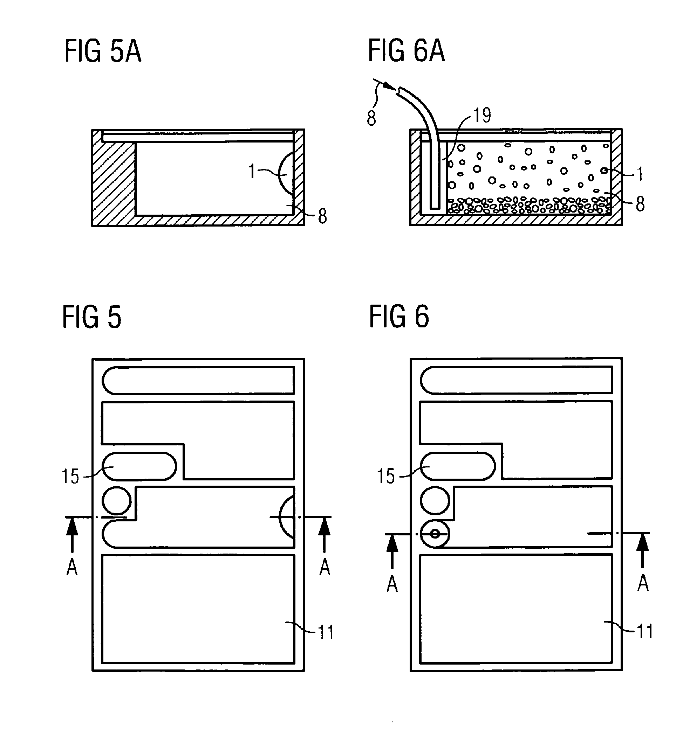

Die Figuren 5, 5A und 6, 6A zeigen Vorratsvarianten für das trocknungsmittel 1. In Fig. 5A ist ein Depot angelegt, in dem beispielsweise über eine semipermeable Membran eine direkte Trocknung eines Reagenz 8 ermöglicht wird. In Fig. 6A sind Reagenz 8 und Trocknungsmittel 1 vermischt und werden erst bei Entnahme des Reagenz 8 getrennt.

## Patentansprüche

1. Verfahren zum Betrieb einer Kartusche (10), die mindestens zwei Behälter (11-17) umfasst, zur Bereitstellung von Reagenzien für ein Biosensorsystem, wobei
- die Reagenzien sowohl in wässriger Lösung, als auch in einem Wasser freien Lösungsmittel fluidisch vorliegen,
- die Reagenzien in den mindestens zwei Behältern jeweils Wasser dicht und Luft dicht zumindest in einem Teilbereich mittels einer Folie (22) verschlossen werden,
- eine Ankopplung an das Biosensorsystem derart geschieht, dass die Folie (22) mit jeweils mindestens einer zum Biosensorsystem zugehörigen Hohlnadel (5) zur Entnahme von Reagenzien durchstochen wird, und
- ein Druckausgleich bei einer Entnahme aus den mindestens zwei Behältern durchgeführt wird, wobei zumindest für die in einem Wasser freien Lösungsmittel aufbereiteten Reagenzien ein Wasser freies Gas eingesetzt wird.

2. Verfahren nach Anspruch 1,
**dadurch gekennzeichnet, dass** mittels eines auf der Kartusche (10) bevorrateten Trocknungsmittels (1) getrocknete Luft erzeugt und mindestens einem der mindestens zwei Behälter (11, 17) zum Druckausgleich zugeführt wird.

3. Verfahren nach Anspruch 1 oder 2,
dadurch gekenntzeichnet, dass das Trocknungsmittel mit mindestens einem Reagenz zu dessen Wasser freier Lagerung vermischt wird.

4. Verfahren nach einem der vorstehenden Ansprüche,
**dadurch gekennzeichnet, dass** das Trocknungsmittel über eine semipermeable Membran von Reagenzien getrennt wird.

5. Verfahren nach einem der vorstehenden Ansprüche,
**dadurch gekennzeichnet, dass** in mindestens einem der mindestens zwei Behälter Reagenzien gekühlt werden.

6. Verfahren nach einem der vorstehenden Ansprüche,
**dadurch gekennzeichnet, dass** zumindest folgende Reagenzien in den jeweiligen Behältern der Kartusche umfasst sind:
- ungekühlte wässrige Lösungen, wie Pufferlösung, Säure oder Lauge,
- gekühlte wässrige Lösungen, wie Enzymlösung, oder
- Reagenzien in Wasser freiem Lösungsmittel, wie Ethylenglycol, Acetonitril, Dimethylsulfoxid, Dioxan, N-Methylpyrrolidon,

7. Verfahren nach einem der vorstehenden Ansprüche,
**dadurch gekennzeichnet, dass** das jeweilige Volumen der mindestens zwei Behälter derart ausgelegt wird, dass die jeweilige Volumen der in den mindestens zwei Behältern befindlichen Reagenzien im Betrieb der Kartusche an dem Biosensorsystem zugleich aufgebraucht sind.

8. Verfahren nach Anspruch 7,
**dadurch gekennzeichnet, dass** das Verhältnis der Volumen der Reagenzien bzw. das der korrespondierenden Behälter, wie
- ungekühlte wässrige Lösung, wie Pufferlösung, Säure oder Lauge, zu
- gekühlter wässriger Lösung, wie Enzymlösung, zu
- Reagenz in wasserfreiem Lösungsmittel, wie Ethylenglycol, Acetonitril, Dimethylsulfoxid, Dioxan, N-Methylpyrrolidon,
in einem Verhältnis von etwa: 900:1:20 vorliegt.

9. Kartusche (10), zur Bereitstellung von Reagenzien für ein Biosensorsystem, umfassend:
- mindestens zwei Behälter (11-17), deren zumindest partiell offene Oberseite mit einer Folie (22) Luft dicht und Wasser dicht verschlossen ist,
- wobei die mindestens zwei Behälter (11-17) einen schräg verlaufenden Boden (21) aufweisen, dessen Gefälle jeweils gleich ausgerichtet ist,
- wobei die Kartusche an ein Biosensorsystem, ankoppelbar ist, indem mindestens eine am Sensorsystem fixierte Hohlnadel (5) durch die Folie (22) des mindestens einen der mindestens zwei Behälter im angekoppelten Zustand hindurchragt und das Ende der Hohlnadel in der Nähe des Bodens des jeweiligen Behälters positioniert ist, und
- auf der Kartusche ein Trocknungsmittel (1) bevorratet ist, zur Darstellung von Wasser freiem Gas.

10. Kartusche nach Anspruch 9,
**dadurch gekennzeichnet, dass** am Boden (21) der mindestens zwei Behälter eine Vertiefung (18) eingelassen ist, zur Darstellung einer tiefsten Stelle am Boden der mindestens zwei Behälter.

11. Kartusche nach einem der Ansprüche 9-10,
**dadurch gekennzeichnet, dass** zumindest Behälter mit Reagenzien mit Wasser freien Lösungsmitteln eine Belüftung mit Wasser freiem Gas aufweisen.

12. Kartusche nach einem der Ansprüche 9-11,
**dadurch gekennzeichnet, dass** das Trocknungsmittel mittel einer Membran (4) begrenzt ist, zur Verhinderung einer Durchmischung mit Reagenzien.

13. Kartusche nach einem der Ansprüche 9-12,
**dadurch gekennzeichnet, dass** bei einer Mischung aus Reagenzien und Trocknungsmitteln ein Gitter (19) vorhanden ist, zur separaten Entnahme von Reagenz.

14. Verwendung einer Kartusche nach einem der Ansprüche 9-13,
- zur Versorgung eines Biosensorsystems beziehungsweise,
- zum Einsatz in einem Verfahren zum Betrieb einer Kartusche nach einem der Ansprüche 1-8,
mit Reagenzien zur Kontrolle von Wasserqualität.

## Claims

1. Method for operating a cartridge (10) which comprises at least two containers (11-17) for the provision of reagents for a biosensor system, wherein
- the reagents are present in fluid form both in aqueous solution and in a water-free solvent,
- the reagents in the at least two containers are sealed, respectively, in water-tight and air-tight manner at least in one partial region by means of a foil (22),
- coupling to the biosensor system is carried out in that the film (22) is pierced with at least one hollow needle (5) associated with the biosensor system for the removal of reagents, and
- a pressure equalization on removal from the at least two containers is carried out, at least for the reagents prepared in a water-free solvent, with a water-free gas.

2. Method according to claim 1, **characterized in that** dried air is generated by means of a drying agent (1) stored on the cartridge (10) and is fed to at least one of the at least two containers (11-17) for pressure equalization.

3. Method according to claim 1 or 2, **characterized in that** the drying agent is mixed with at least one reagent for water-free storage of said reagent.

4. Method according to one of the preceding claims, **characterized in that** the drying agent is separated from reagents by means of a semi-permeable membrane.

5. Method according to one of the preceding claims, **characterized in that** reagents are cooled in at least one of the at least two containers.

6. Method according to one of the preceding claims, **characterized in that** at least the following reagents are included in the respective containers of the cartridge:
- non-cooled aqueous solutions such as buffer solution, acid or alkali,
- cooled aqueous solutions such as enzyme solution, or
- reagents in water-free solvents, such as ethylene glycol, acetonitrile, dimethyl sulfoxide, dioxane, N-methylpyrrolidone.

7. Method according to one of the preceding claims, **characterized in that** the respective volumes of the at least two containers are configured such that, during operation of the cartridge, the respective volumes of the reagents held in the at least two containers are used up simultaneously in the biosensor system.

8. Method according to claim 7, **characterized in that** the ratio of the volumes of the reagents and of the corresponding containers, such as
- non-cooled aqueous solution such as buffer solution, acid or alkali, to
- cooled aqueous solution, such as enzyme solution, to
- reagent in water-free solvent, such as ethylene glycol, acetonitrile, dimethyl sulfoxide, dioxane, N-methylpyrrolidone,
is approximately 900 : 1 : 20.

9. Cartridge (10) for providing reagents for a biosensor system, comprising:
- at least two containers (11-17), the at least partially open upper sides of which are each sealed in an air-tight and water-tight manner by means of a foil (22),
- the at least two containers (11-17) having an inclined base (21), the slopes of which are aligned identically,
- wherein the cartridge can be coupled to a biosensor system in that at least one hollow needle (5) which is fixed at the sensor system extends through the foil (22) of at least one of the at least two containers in the coupled state and the end of the hollow needle is positioned in the vicinity of the base of the respective container, and
- a drying agent (1) is stored at the cartridge for generating water-free gases.

10. Cartridge according to claim 9, **characterized in that** a depression (18) is set into the base (21) of the at least two containers to form a deepest site at the base of the at least two containers.

11. Cartridge according to one of the claims 9-10, **characterized in that** at least containers containing reagents having water-free solvents have ventilation with water-free gas.

12. Cartridge according to one of the claims 9-11, **characterized in that** the drying agent is delimited by a membrane (4) for preventing mixing with reagents.

13. Cartridge according to one of the claims 9-12, **characterized in that** in the case of a mixture of reagents and drying agents, a grid (19) is present for separate removal of reagent.

14. Use of a cartridge according to one of the claims 9-13 for supplying a biosensor system with reagents or for use in a method for operating a cartridge according to one of the claims 1-8 for monitoring water quality.

## Revendications

1. Procédé de fonctionnement d'une cartouche (10) comprenant au moins deux réservoirs (11-17), destinée à fournir des réactifs pour un système biocapteur,
- les réactifs étant présents sous forme de fluide aussi bien dans une solution aqueuse que dans un solvant déshydraté,
- les réactifs étant enfermés dans les au moins deux réservoirs de manière étanche à l'eau et à l'air au moins dans une partie au moyen d'une feuille (22),
- un couplage au système biocapteur étant opéré de telle façon que la feuille (22) est transpercée avec au moins une aiguille creuse (5) faisant partie du système biocapteur pour prélever des réactifs, et
- lors d'un prélèvement dans les au moins deux réservoirs une compensation de pression étant effectuée, en utilisant, au moins pour les réactifs préparés dans un solvant déshydraté, un gaz déshydraté.

2. Procédé selon la revendication 1
**caractérisé en ce que**
au moyen d'un agent déshydratant (1) approvisionné sur la cartouche (10), de l'air séché est produit et amené au moins à l'un des au moins deux réservoirs (11, 17) pour la compensation de pression.

3. Procédé selon la revendication 1 ou 2,
**caractérisé en ce que** l'agent déshydratant est mélangé avec au moins un réactif en vue du stockage de ce dernier à l'état déshydraté.

4. Procédé selon l'une des revendications précédentes,
**caractérisé en ce que** l'agent déshydratant est séparé des réactifs au moyen d'une membrane semi-perméable.

5. Procédé selon l'une des revendications précédentes, **caractérisé en ce que** les réactifs sont refroidis dans au moins l'un des au moins deux réservoirs.

6. Procédé selon l'une des revendications précédentes, **caractérisé en ce qu'**au moins les réactifs suivants sont contenus dans les réservoirs respectifs de la cartouche :
- des solutions aqueuses non refroidies, telles qu'une solution tampon, un acide ou une base,
- des solutions aqueuses refroidies, telles qu'une solution enzymatique, ou
- des réactifs dans un solvant déshydraté, tels que l'éthylèneglycol, l'acétonitrile, le diméthylsulfoxyde, le dioxane, le N-méthylpyrrolidone.

7. Procédé selon l'une des revendications précédentes, **caractérisé en ce que** le volume respectif des au moins deux réservoirs est étudié de telle sorte qu'en fonctionnement de la cartouche sur le système biocapteur les volumes respectifs des réactifs se trouvant dans les au moins deux réservoirs sont épuisés en même temps.

8. Procédé selon la revendication 7,
**caractérisé en ce que** le rapport volumique des réactifs, respectivement celui des réservoirs correspondants, comme
- une solution aqueuse non refroidie, telle qu'une solution tampon, un acide ou une base, par rapport à
- une solution aqueuse refroidie, telle qu'une solution enzymatique, par rapport à
- un réactif dans un solvant déshydraté, tel que l'éthylèneglycol, l'acétonitrile, le diméthylsulfoxyde, le dioxane, le N-méthylpyrrolidone,
est égal à environ 900:1:20.

9. Cartouche (10) destinée à fournir des réactifs pour un système biocapteur, comprenant :
- au moins deux réservoirs (11-17), dont le côté supérieur au moins partiellement ouvert est obturé de manière étanche à l'air et à l'eau par une feuille (22),
- les au moins deux réservoirs (11-17) ayant un fond (21) incliné, dont la pente est à chaque fois orientée dans le même sens,
- la cartouche pouvant être couplée à un système biocapteur par le fait qu'au moins une aiguille creuse (5) fixée sur le système capteur traverse la feuille (22) d'au moins l'un des deux réservoirs à l'état couplé et que l'extrémité de l'aiguille creuse est positionnée à proximité du fond du réservoir respectif, et
- un agent déshydratant (1) étant approvisionné sur la cartouche (10), pour obtenir un gaz déshydraté.

10. Cartouche selon la revendication 9,
**caractérisée en ce qu'**un creux (18) est ménagé au fond (21) des au moins deux réservoirs, pour obtenir un point le plus bas au fond desdits au moins deux réservoirs.

11. Cartouche selon l'une des revendications 9-10,
**caractérisée en ce qu'**au moins les réservoirs contenant des réactifs avec des solvants déshydratés bénéficient d'une aération avec un gaz déshydraté.

12. Cartouche selon l'une des revendications 9-11,
**caractérisée en ce que** l'agent déshydratant est limité au moyen d'une membrane (4), pour empêcher un mélange avec les réactifs.

13. Cartouche selon l'une des revendications 9-12, **caractérisée en ce que** dans le cas d'un mélange de réactifs et d'agents déshydratants une grille (19) est prévue, pour le prélèvement séparé de réactifs.

14. Utilisation d'une cartouche selon l'une des revendications 9-13,
- pour l'alimentation d'un système biocapteur, respectivement
- pour la mise en oeuvre dans un procédé de fonctionnement d'une cartouche selon l'une des revendications 1-8,
avec des réactifs pour le contrôle de la qualité de l'eau.
